Europäisches Patentamt

European Patent Office

Office européen des brevets

(11) Publication number: **0 088 382**
**B1**

(12) **EUROPEAN PATENT SPECIFICATION**

(45) Date of publication of patent specification: **19.06.85**

(21) Application number: **83102098.7**

(22) Date of filing: **03.03.83**

(51) Int. Cl.⁴: **C 07 C 139/14,**
**C 07 C 143/22, A 61 K 9/14**

(54) **Stabilization of azulene derivatives.**

(30) Priority: **09.03.82 JP 37410/82**

(43) Date of publication of application:
**14.09.83 Bulletin 83/37**

(45) Publication of the grant of the patent:
**19.06.85 Bulletin 85/25**

(84) Designated Contracting States:
**DE FR GB IT**

(56) References cited:
**DE-B-1 034 327**
**GB-A-1 422 974**
**GB-A-2 004 182**

(73) Proprietor: **NIPPON SHINYAKU COMPANY,**
**LIMITED**
**14, Kisshoin Nishinosho Monguchicho**
**Minami-ku Kyoto-shi Kyoto (JP)**

(72) Inventor: **Kawamata, Masanobu**
**17-6, Kitanakaincho Nisoninmonzen Saga**
**Ukyo-ku Kyoto 616 (JP)**
Inventor: **Ushimaru, Koichi**
**17 Muromachi Nishiiru Kamitachiuridori**
**Kamikyo-ku Kyoto 602 (JP)**
Inventor: **Goshi, Hiroyuki**
**14-14 Kitayamadai-1-chome**
**Koseicho Shiga Prefecture 520-32 (JP)**
Inventor: **Miyasako, Hideichi**
**684-56 Harimadacho Moriyamashi**
**Shiga Prefecture 524 (JP)**

(74) Representative: **Wey, Hans-Heinrich, Dipl.-Ing.**
**et al**
**Patentanwälte Müller-Börner & Wey**
**Widenmayerstrasse 49**
**D-8000 München 22 (DE)**

Courier Press, Leamington Spa, England.

## 0 088 382

**Description**

The present invention relates to a method of stabilizing sodium 1,4-dimethylisopropylazulene-3-sulfonate (abbreviated as GASN as hereinafter) which is a water soluble derivative of guaiazulene.

GASN exhibits antiinflammatory and anti-ulcer actions and is widely used as a pharmaceutical in a form of tablets or granules. Though GASN is soluble in warm water, alcohol and acetic anhydride, it is sparingly soluble in cold water and is nearly insoluble in ether and benzene. Therefore, it is usually purified by recrystallizing from water. The crystals obtained by recrystallizing from water are needles or flakes.

GASN is an unstable compound even in a solid state and decomposed into sublimate substance and pasty substance even at room temperatures. Thus it is not possible to store it for long time in preparation forms such as tablets and granules.

Therefore, it has been attempted that antioxidants and weakly basic metals salts or alkali earth metal are added to stabilize GASN against heating (cf. German Patent No. 1,034,327) or, recently, that amino acid is added to stabilize GASN against heating (cf. Japanese Patent Publication Sho-49-11219). Unfortunately there are several disadvantages in those methods such as that addition of stabilizer is necessary or the resulting effect is not always satisfactory.

During the course of studies on GASN granular preparation, the present inventors have unexpectedly found that, if the manufacture of the preparation is conducted under a definite condition, stable GASN preparation is obtained without the use of stabilizers and, after extensive studies thereafter, the present inventors have accomplished the present invention.

The present inventive method for stabilization is quite simple in that GASN is dissolved in a solvent and from the resulting solution is promptly removed the solvent by the method which is further illustrated hereinafter.

Thus, GASN is dissolved in water, lower aliphatic alcohols, or aqueous solutions of such alcohols and then the solvent is removed therefrom by a quick method, i.e. spray-drying or by lyophilization. Examples of lower aliphatic alcohols applicable are straight chained or branched alcohols with one to four carbon atoms and, among them, methyl alcohol or ethyl alcohol is preferred. When such alcohols are used as aqueous solutions thereof, the concentration is not particularly restricted.

One of the most important features of the present invention is the method of evaporating the solvent. Thus, spray-drying or lyophilization which has not been applied as a method for stabilization is applied in the present invention and a solute which is stable against heating is obtained as a result of evaporating the solvent. Being based on such a novel finding, the present invention has been accomplished.

When the solvent is removed under ordinary pressure or by such an apparatus as a rotary evaporator in vacuo, the solution is concentrated and a part of the solute appears as crystals. But, there is no hope of obtaining stabilized solute by such methods. The present invention is characterized in the use of a quick method for removing or separating the solvent by spray-drying or by lyophilization.

Powder X-ray diffraction of GASN obtained by the present invention does not show particular peaks as compared with that of crystals and it is obvious that the product is in non-crystalline form. This is one of the most important characteristic features of GASN obtained by the present invention method.

Concrete examples according to the present invention are given as hereunder and stabilization effect will be shown.

Example 1.

GASN (50 grams) is dissolved in 5 liters of water at 25°C and subjected to a spray-drying using a mobile minor type spray drier (Niro Company) at a spray drying rate of 2 liters per one hour (temperature of supplied air is 100°C) to give finely powdery GASN.

Example 2.

GASN (50 grams) is dissolved in 5 liters of water at 25°C, 4950 grams of lactose is subjected to flowing at the condition of air in-taking temperature of 70°C using a fluidized bed grain manufacturing apparatus WSG-5 (Gratt-Okawara), and the above solution is sprayed at 100 ml/minute and dried to give a composition containing 1% GASN.

Example 3.

GASN (50 grams) is dissolved at room temperatures in 3.5 liters of 50% methanol and treated in the same way as in Example 2 (air intaking temperature: 60°C) to give a composition of GASN:lactose=1:99.

Example 4.

GASN (1 gram) is dissolved in 100 ml of water and subjected to lyophilization by a lyophilizer (DC-35, Yamato Co) using actone-dry ice as a freezing mixture.

GASN obtained in Examples 1 to 4 are allowed to stand at 40°C after being made into the samples as illustrated later and the residual GASN is measured by the formula

# 0 088 382

$$\text{Amount of GASN (mg)}=\frac{A}{20.25}\times 1000$$

by observing an extinction at 568 nm in a phosphate buffer of pH 7.0. The stability data after storing at 40°C are given in Table 1.

Method of manufacturing samples are as follows.

Sample 1: One gram of GASN obtained in Example 1 is mixed with 99 grams of lactose to make 100 grams.

Sample 2: The composition obtained in Example 2 is used as it is.

Sample 3: The composition obtained in Example 3 is used as it is.

Sample 4: One gram of GASN obtained in Example 4 is mixed with 99 grams of lactose to make 100 grams.

Control: One gram of untreated GASN is mixed with 99 grams of lactose to make 100 grams.

Each sample is placed in Petri dishes and the upper part is tightly closed with polyethylene film.

TABLE 1.
Residual rate (in %) of GASN treated at 40°C

| Samples | Immediately after manufg | 1 Week | 2 Weeks | 3 Weeks | 4 Weeks after |
|---------|--------------------------|--------|---------|---------|---------------|
| 1       | 100                      | 97.9   | 91.4    | 80.3    | 68.6          |
| 2       | 100                      | 98.9   | 97.1    | 94.1    | 92.6          |
| 3       | 100                      | 97.8   | 95.3    | 93.4    | 90.4          |
| 4       | 100                      | 98.1   | 79.6    | 54.1    | 28.7          |
| Control | 100                      | 26.3   | 3.5     | 0       | 0             |

(Qualitative data immediately after manufacturing are set 100)

It is obvious from Table 1 that, as compared with the control, GASN obtained by the present invention method is higher in stabilization.

In the above methods, addition of inert or active soluble additives to GASN solutions followed by treating similarly will not destroy the stabilization of GASN so far as the above given rush removal of solvent is conducted. It is incidentally found that the stability of GASN compositions to which insoluble additives are added is nearly the same as that of the compositions of Examples 2 and 3.

## Claims

1. A method of manufacturing sodium 1,4-dimethylisopropylazulene-3-sulfonate which is stable against heating, characterized in that, a solvent is removed or separated from a solution of sodium 1,4-dimethylisopropylazulene-3-sulfonate by spray-drying or lyophilization.

2. A method according to Claim 1 in which the solvent is water, methyl alcohol, ethyl alcohol or a mixture thereof.

## Patentansprüche

1. Verfahren zur Herstellung von hitzestabilem Natrium-1,4-dimethylisopropylazulen-3-sulfonat, dadurch gekennzeichnet, daß aus einer Lösung von Natrium-1,4-dimethylisopropylazulen-3-sulfonat durch Sprühtrocknung oder Lyophilisierung das Lösungsmittel entfernt wird.

2. Verfahren nach Anspruch 1, bei dem das Lösungsmittel Wasser, Methanol, Äthanol oder ein Gemisch daraus ist.

## Revendications

1. Procédé de fabrication du 1,4-diméthylisopropyl-azulène-3-sulfonate de sodium stable à la chaleur, caractérisé en ce que l'on élimine ou sépare un solvant d'une solution de 1,4-diméthylisopropyl-azulène de sodium par séchage par projection ou par lyophilisation.

2. Procédé selon la revendication 1, caractérisé en ce que le solvant est l'eau, le méthanol, l'éthanol ou un de leurs mélanges.

3